# EUROPEAN PATENT APPLICATION

(11) **EP 4 667 063 A1**
(43) Date of publication of application: **24.12.2025**
(21) Application number: 24305956.5
(22) Date of filing: 18.06.2024
(51) Int. Cl.: A63B 21/00, A63B 23/04, A61B 5/00, A61B 5/22, A61B 5/11, G09B 19/00, A63B 24/00, A63B 71/06

(54) **ACCESSORY FOR EQUIPPING A TRAINING MACHINE WITH A MEASURING UNIT**

(71) Applicant: Kinvent Biomecanique, 34000 Montpellier (FR)
(72) Inventor: KOLLIAS, Athanase, 34170 CASTELNAU-LE-LEZ (FR); ATHANASSOPOULOS, Emmanouil, 34000 MONTPELLIER (FR); KOTOPOULOS, Athanasios, 54643 THESSALONIKI (GR)
(74) Representative: IPAZ

(57) **Abstract**

The invention relates to an accessory (100) for equipping a training machine with a measuring unit (122), the accessory comprising:
- a body with a rear face and a front face, said rear face being intended to bear against a surface of the training machine on which a user applies a pressure, said front face being intended to receive said measuring unit (122),
- at least one first attachment means (104, 105, 108, 118) for attaching said body to said training machine, and
- at least one second attachment (106) means for attaching said measuring unit (122) to said body;
so that when a user applies a pressure to said measuring unit (122), the pressure is transmitted to the surface of the training machine, the measuring unit (122) being able to measure at last one data relating to said pressure.

## Description

### Field of the invention

The present invention relates to an accessory for equipping a training machine with a measuring unit. The present invention further relates to an apparatus and a training machine equipped with such an accessory.

The field of the invention is the field of accessories for equipping a training machine with a measuring unit, and more generally the field of the training machines.

### Background

In general, training machines, whether in a gym or a rehabilitation centre for example, do not make it possible to measure the effort made by the user during use.

However, practitioners, such as doctors or physiotherapist, may need to measure the effort of a person during a diagnosis, or a period of rehabilitation. Trainers may further need to use this kind of connected training machines for measuring the performance of an athlete, and optionally monitoring said performance over time during a training period for example.

There are many measuring apparatus intended to be, for example, attached to a surface of a training machine to overcome this problem. However, the training machines are not all similar, they each have their own technical features, which does not always make it possible to easily attach a measuring apparatus to them. In addition, they are bulky and not easy to ca rry.

Despite all efforts, current measuring apparatus are not adapted to all training machines. This yields either to uncomfortable use of the machine for some users, or to the use of different devices for one machine.

Another possibility is to provide connected training machines that make it possible to measure the effort made by the user, but this kind of machines are excessively expensive.

A purpose of the present invention is to overcome at least one of these drawbacks.

Another purpose of the present invention is to provide a solution for equipping a training machine with a measuring unit that is adapted to be attached on a surface of different types of training machines.

Another purpose of the present invention is to provide a solution for equipping a training machine with a measuring unit that is lightweight, allowing portability and ease of mounting.

Another purpose of the present invention is to provide an accessory for equipping a training machine with a measuring unit, the measuring unit providing more accurate results.

### Summary of the invention

The invention makes it possible to achieve at least one of these aims by an accessory for equipping a training machine with a measuring unit, the accessory comprising:
- a body with a rear face and a front face, said rear face being intended to bear against a surface of the training machine on which a user applies a pressure, said front face being intended to receive said measuring unit,
- at least one first attachment means for attaching said body to said training machine, and
- at least one second attachment means for attaching said measuring unit to said body;
so that when a user applies a pressure to said measuring unit, the pressure is transmitted to the surface of the training machine, the measuring unit being able to measure at last one data relating to said pressure.

The present invention thus proposes an accessory comprising at least one first attachment means to attach the accessory on a surface of training machine. The at least one first attachment means allow the accessory to adapt to different training machine surfaces. Moreover, the accessory according to the invention comprises at least one second attachment means to attach a measuring unit on the body of said accessory. The at least one second attachment means makes it easy and simple to attach the measuring unit to the accessory.

Furthermore, when attached to an inclined surface, the accessory is positioned in a manner that ensures the force or pressure exerted on the measuring unit, which is connected to the accessory, is not purely vertical. This consideration allows for accurate measurement and avoids potential deviations caused by vertical forces.

Overall, the accessory offers adaptability, portability, and optimal functionality, making it a versatile solution for various training machine surfaces and ensuring reliable and precise measurements.

In the present document, "mounting element", or "fixing means", designates any type of fastener for attaching the measuring unit on the front face of the body such as screws, nails, nuts, bolts, and others.

In the present document, "strap" designates a strap or strip of rigid fabric.

In the present document, "flexible rubber attachment" designates more particularly, a flex disc for example.

In the present document, "training machine" designates a press-type training machine, more particularly, a fitness or medical equipment designed to target and strengthen various muscles of the upper body, particularly the chest, shoulders, and arms. The user sits or lies on the machine and performs pressing movements, pushing against the resistance provided by weights, hydraulic systems, or other means. The training machine may also designate a leg press-type training machine, more particularly, a fitness or medical equipment specifically designed to target and strengthen the muscles of the lower body, primarily the quadriceps, hamstrings, and glutes. It typically consists of a seat or platform that the user sits or lies on, along with a footplate or sled connected to a resistance system.

According to some embodiments, the at least one second attachment means may comprise at least one mounting hole to fix the measuring unit on the front face of the body.

In some embodiments, at least one mounting hole may contain at least one flexible rubber attachment and at least one screw with a pre-set depth cut.

This feature enables the application of a constant preload on the accessory when the user applies pressure to the measuring unit. The constant preload allows the surface to flex appropriately under load and self-align, ensuring accurate measurements without compromising the calibration or introducing additional loads.

In some embodiments, the at least one second attachment means may comprise at least one hole or thread provided in the body and intended to receive at least one mounting element for securing the body to the surface of the training machine if the latter is configured to receive said mounting element.

In some embodiments, the at least one first attachment means may comprise at least one strap equipping the body and provided to attach said body to the surface of the training machine.

This makes it possible to attach the accessory to a surface that does not make it possible to receive screws, for example. This feature makes it possible attaching the accessory on any type of training machine, regardless the architecture of said machine. Indeed, the length and/or the width of the straps may be adapted easily as a function of the training machine without modifying the accessory. It is even possible associating several straps to the accessory and using the one adapted to each machine, depending on the concerned training machine.

The at least one strap may be secured to the rear face, or to the front face, or may be inserted in the body in a channel provided in said body so that it extends around said body.

The at least one strap may be in a single piece.

The at least one strap may be realised in several pieces, each attached to the body.

At least one strap may be equipped with at least one ratcheting mechanism.

The at least one ratcheting mechanism allows the strap to be adjusted to the surface of the training device, and therefore to keep the accessory in place.

The at least one strap may be equipped with another attaching means, such as, for example, scratch, reusable double-sided adhesive, press button(s), etc.

In some embodiments, the at least one first attachment means may comprise at least one magnet, for example arranged in/on the body, and more particularly on the rear face of the body.

Advantageously, each magnet may possess a pulling force of over 30kg, enabling instant attachment to ferromagnetic surfaces.

In some embodiments, the at least one first attachment means may comprise at least one reusable double-sided adhesive arranged on the rear face of the body.

The rear side of the accessory may be coated with reusable double-sided adhesive to induce higher friction and adhesion.

This reusable double-sided adhesive may be easily removed, cleaned with water and reused on any surface.

The at least one reusable double-sided adhesive may be comprise, or correspond to, a nanotape.

According to some embodiments, the at least one first attachment means may comprise at least one mounting peg intended to be inserted through the body and to be attached on the surface of the training machine if the latter is configured to receive said mounting peg.

According to some embodiments, the at least one first attachment means may comprise at least one suction cup intended to be used with a used with a manual or electronic pump to attach on the surface of the training machine if the latter has a flat or textured surface.

In some embodiments, the body may be made of a single piece.

The single piece may be made of sturdy material.

In some embodiments, the body of the accessory according to the invention may comprise a stacked assembly of at least two sheets.

At least one, in particular each, of the sheets, may be made of sturdy material.

In some embodiments, the body of the accessory may comprise:
- a front sheet,
- a rear sheet, and
- at least one intermediary sheet, and in particular two intermediary sheets, stacked between said front and rear sheets.

The accessory may comprise a robust stacked assembly of sheets made from durable materials. This design enables one or more straps to pass through the sheets.

In some embodiments of the body, two intermediary sheets may be positioned side by side between the rear and front sheets. They create a channel between them, specifically designed to accommodate an attaching strap that crosses the body. This configuration ensures efficient and secure attachment of the accessory.

In some embodiments, at least one sheet according to the invention may be made of aluminium or plastic or carbon Fiber composite materials.

According to another aspect of the present invention, it is proposed an apparatus comprising:
- at least one accessory according to the invention, and
- at least one measuring unit attached on the front face of the body of said accessory.

In some embodiments, the measuring unit may comprise:
- a communication module for communicating with a distant computer/server, in a wireless or wired fashion;
- at least one force sensor,
- at least one inertial sensor.

Of course, the measuring unit may comprise a force sensor, especially for measuring the force or pressure exerted by the user. Such a sensor is known and its architecture, and its integration in the measuring unit, may be done according to known techniques.

The measuring unit may also comprise an angle sensor, and/or an accelerometer.

Such sensor(s) allow controlling, and proper positioning, of the device during use, enhancing the usability of the measuring unit according to the invention, and the accuracy of the measurements.

Plus, such sensor(s) allows efficient and proper use of the measuring unit according to the invention for measurement(s)/exercise(s) involving movement of the hand and/or of the foot and/or the third person, such as isokinetic measurement(s)/exercise(s), for example when the user has to press on his feet during the exercise.

Such sensor(s) also allows controlling and proper positioning of the measuring device when the user has to exert force at a given angle.

According to another aspect of the present invention, it is proposed a training machine comprising at least one accessory according to the invention, the accessory intended to be attached against a surface of the training machine.

According to another aspect of the present invention, it is proposed a training machine comprising at least one measuring apparatus according to the invention, the measuring apparatus being configured to be attached on a surface of the training machine.

In some embodiments, the training machine may be a leg press, and the accessory is intended to be attached to a surface of said machine provided to be pushed by the leg(s) of a user.

Of course, this example is in no way limitative and the machine may be any type of training machine having a surface on which a user applies an effort.

### Description of the figures and embodiments

Other advantages and characteristics will become apparent on examination of the detailed description of an embodiment which is in no way limitative, and the attached figures, where:
- FIGURE 1 is a representation of a non-limitative example of an accessory according to the invention, according to an exploded view;
- FIGURE 2 is a diagrammatic representation of the front side of the accessory of the Figure 1;
- FIGURE 3 is a diagrammatic representation of the rear side of the accessory of the FIGURE 1;
- FIGURE 4 is a representation of a non-limitative example of a measuring apparatus according to the invention; according to an exploded view; and
- FIGURE 5 is a representation of a non-limitative example of a training machine equipped with measuring apparatus according to the invention.

It is well understood that the embodiments that will be described below are in no way limitative. In particular, it is possible to imagine variants of the invention comprising only a selection of the characteristics described hereinafter, in isolation from the other characteristics described, if this selection of characteristics is sufficient to confer a technical advantage or to differentiate the invention with respect to the state of the prior art. Such a selection comprises at least one, preferably functional, characteristic without structural details, or with only a part of the structural details if this part alone is sufficient to confer a technical advantage or to differentiate the invention with respect to the prior art.

In the FIGURES, elements common to several figures retain the same reference.

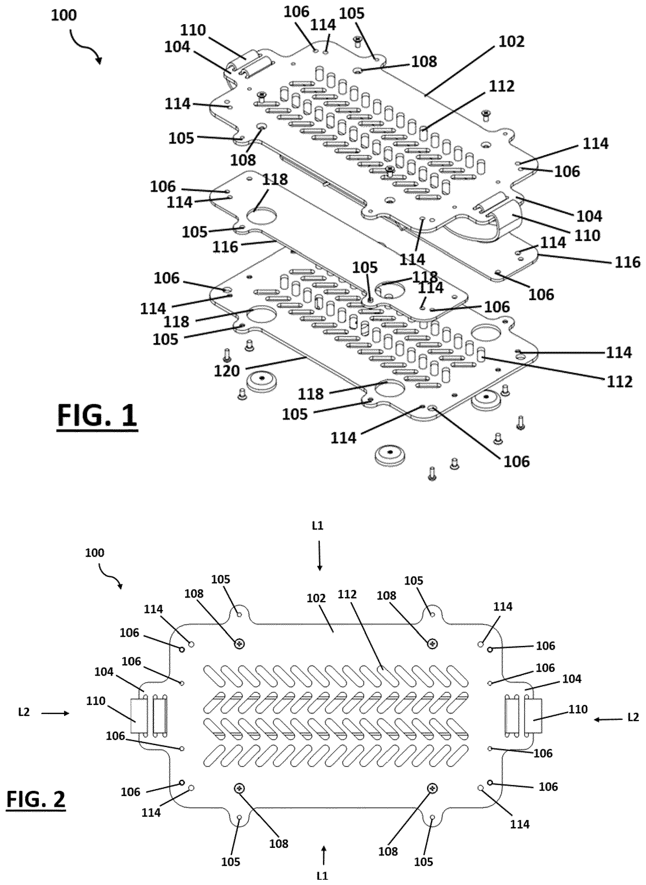

FIGURE 1 is a representation of a non-limitative example of an accessory according to the invention, according to an exploded view.

FIGURE 2 is a diagrammatic representation of the front side of the accessory of Figure 1.

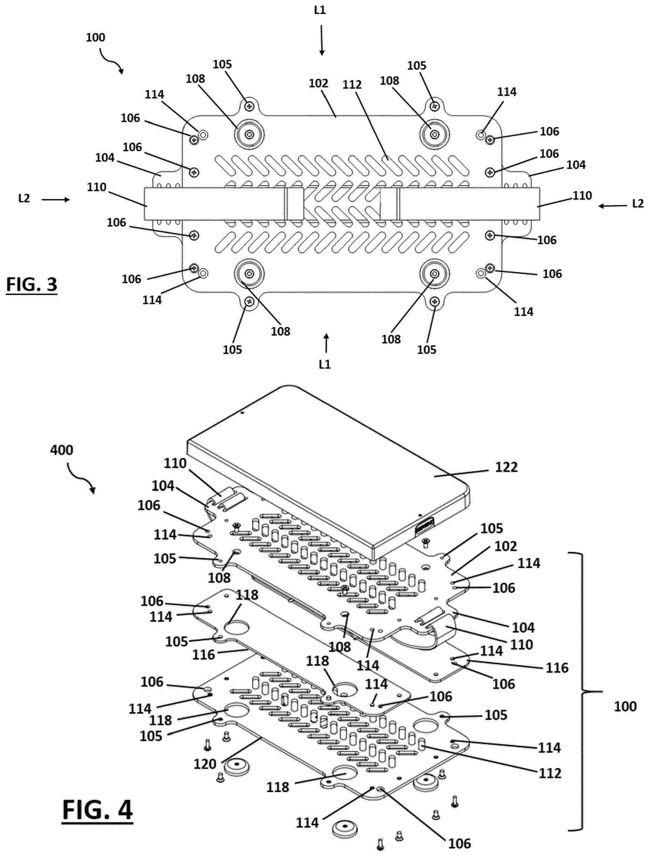

FIGURE 3 is a diagrammatic representation of the rear side of the accessory of FIGURE 1.

The accessory 100, shown in FIGURES 1-3, serves the purpose of attaching a measuring unit to its front face while being securely fastened to a training machine surface at its rear face. When the user applies pressure to the measuring unit, this force is transmitted to the surface of the training machine. Consequently, the measuring unit is capable of capturing and measuring at least one data item related to the applied pressure, and/or angle and/or velocity and more generally to the effort applied by the user during the exercise.

In the non-limitative example shown on FIGURES 1-3, the accessory 100 has a general rectangular shape. Of course, in other examples the accessory may also be designed in various other shapes, such as oval, square, or other general forms. The choice of the shape depends on specific design considerations.

The accessory 100 may be constructed from a wide range of materials suitable for attaching a measuring unit to a training machine, and more particularly suitable for resisting to the applied effort during the exercise. For instance, plastic, particularly hard plastic, or aluminium, or carbon fibre composite laminate may be used for manufacturing the accessory 100. The material selection may be based on factors like durability, strength, weight, and compatibility with the intended application.

In the non limitative example of FIGURES 1-3, the accessory 100 comprises a body comprising a stacked assembly of two main sheets 102, 120 and two intermediary sheets 116 positioned between the two main sheets 102, 120.

The sheet 102 comprises two lateral sides, designated as L1 and two bottom sides, designated as L2. The sheet 102 comprises a first attachment means 104 may use to attach the accessory to a training machine surface. The first attachment means 104 correspond to a tab projecting from each part of the bottom, L2, of the sheet 102. Each tab comprises three slots for example, for passing a strap 110. The three slots are parallel to one another and equidistantly spaced. In a preferred embodiment, the slots correspond to friction slots raised on the sides. The friction slots on the sides provide force multiplication to prevent slippage and generate a perpendicular force that helps secure the accessory in place. The first attachment means 104 are symmetrically distributed on both sides, L2, of the sheet 102. Of course, in other embodiments, the tabs may be positioned elsewhere, for example on both sides, L1. The number of slots is not limitative.

The strap 110 can be crafted from various materials that are well-suited for attaching an accessory to a training machine. Examples of suitable materials for the strap 110 include polypropylene and polyester. These materials offer durability, strength, and flexibility.

On each of these sides L1, sheet 102 comprises another first attachment means 105. Each first attachment means 105 consist of a distributed holes positioned on protruding ears located at the periphery of the sheet 102. The first attachment means 105 are symmetrically distributed on both sides, L1, of the sheet 102. The first attachment means 105 are configured to receive a fixation means such as screws. In other embodiments, the first attachment means 105 may be may be positioned elsewhere on the sheet 102.

Additionally, the sheet 102 includes second attachment means 106 on each side of the bottom L2 that facilitate the secure attachment of the measuring unit to the front face of the accessory 100. Each side L2 comprises mounting holes, more exactly four mounting holes, arranged in a line parallel to the lateral side L2 of the sheet 102. The second attachment means 106 are symmetrically distributed on both sides, L2, of the sheet 102. The second attachment means 106 are configured to receive a mounting elements such as screws. Of course, in other embodiments, the second attachment means 106 may be positioned elsewhere on the sheet 102. The number of mounting holes is not limitative. In a preferred embodiment, the second attachment means 106 comprise a flexible rubber attachment and one screw with a pre-set depth cut.

The sheet 102 also comprises openings configured to fix another first attachment means 108 that correspond to magnets and/or suction cups. Magnets are fixed on the sheet 102 with fixation means, such as screws for example via openings. The openings 108 are symmetrically distributed on both sides, L1, of the sheet 102. The accessory 100 comprises magnets, more particularly four magnets, two magnets being positioned on the body along each lateral side L1. Of course, in other embodiments, the numbers of magnets may be different and the openings 108 may be positioned elsewhere on the sheet 102.

The sheet 102 also comprises another opening 114 corresponding to holes, more particularly four holes positioned at each corner of the sheet 102.

In addition, the sheet 102 comprises openings 112 in its central part. In another embodiment, the sheet 102 may not comprise openings 112.

Sheet 120 is similar to sheet 102. Sheet 120 has the same features that sheet 102, however, sheet 120 includes openings 118 with a diameter suitable for accommodating a magnet or a suction cup instead of openings 108. The openings 118 have a diameter equal to that of a magnet or a suction cup. Sheet 120 does not include a first attachment means 104. Two of the orifices 106 are designed with a larger diameter compared to the other two, enabling the passage of a fixing means. This asymmetry in the orifice sizes facilitates the attachment of the accessory using suitable fixing means.

The two intermediary sheets 116 exhibit symmetry and share the same shape as the lateral sides L1 of sheet 102. Each intermediary sheet 116 is predominantly rectangular, mirroring the shape of sheet 102. However, in alternative embodiments, the shape of intermediary sheets 116 may differ from that of sheet 102 and/or sheet 120. This design flexibility allows for variations in the overall shape and configuration of the accessory, accommodating different functional or aesthetic requirements.

Each sheet 116 comprises:
- first attachment means 105, positioned in a manner similar to that of sheet 102.
- second attachment means 106 that facilitate the secure attachment of the measurement sensor to the front face of the accessory as previously described.
- openings 118 intended to accommodate a magnet and/or a suction cup. The openings 118 are positioned to overlap with the openings 108 and 118 of main sheets 102 and 120. The openings 118, when superimposed, create a space for accommodating the magnet and/or a suction cup within said sheets.
- opening 114 corresponding to holes, more particularly four two positioned at each corner of the sheet 102, more particularly on the lateral sides L1.

The intermediary sheets 116 are positioned between sheets 102 and 120 so that the first attachment means 106 and corresponding openings 108, 118 align. intermediary sheets 116 are spaced apart from each other between the two sheets 102 and 120 to allow the passage of a strap.

When the accessory is mounted, the strap 110 is positioned in the middle of the two intermediary sheets 116 and between sheets 102 and 120. The intermediary sheets 116 are designed to accommodate the strap 110 within the body of accessory 100. The strap 110 is then threaded through the slots of the tabs 104 at each of its ends. The magnets and/or the suction cups are positioned in openings 118 and are each secured by a screw through openings 108. The screws passing through the orifices 106 are configured to fix a measuring unit positioned on the front face of the body 102 (not visible in the figures).

Therefore, in this embodiment, the accessory 100 includes four different first attachment means:
- the magnets 108,
- the strap(s) 110,
- the holes 105
- the suction cups.

There is an additional first attachment means (not visible in the figures) that corresponds to reusable double-sided adhesive or pieces of reusable double-sided adhesive. These pieces of reusable double-sided adhesive are arranged on the rear face of the sheet 120, such as on the lateral sides L2. Alternatively, in other embodiments, the pieces of reusable double-sided adhesive can be positioned on any section of the rear side of the sheet 120. The reusable double-sided adhesive allows for easy and quick attachment of the accessory 100 to a training machine's surface.

In one embodiment, if the surface of the training machine is designed to accommodate magnets, such as a steel surface, the accessory 100 can be attached by placing its rear face against the surface. The magnets will then securely adhere to the steel surface.

In another embodiment, the accessory 100 can be attached to the training machine's surface using a strap. The accessory 100 is positioned on the surface, and the two ends of the strap are looped around the surface. Subsequently, the two ends are fastened together behind the surface. In a preferred embodiment, a ratcheting mechanism is used to secure the two ends together. The ratcheting mechanism corresponds to conventional and well-known methods used for attaching straps. In other embodiments, the two ends of the straps can be fixed by other means such as: snaps, scratches, double-sided adhesive tape or any other means making it possible to fix the straps.

In another embodiment, the accessory 100 can be attached to the surface of the training machine using the provided holes 105. The surface of the training machine must be designed to accommodate fixation means, such as screws. The accessory 100 is positioned underneath the surface, and a user securely fastens it by inserting mounting peg or screws through the designated holes 105. This ensures a stable and reliable attachment between the accessory and the training machine surface.

In another embodiment, if the surface of the training machine is designed as flat or slightly curved, the accessory 100 can be attached by placing its rear face against the surface. The suction cups will then securely adhere to the steel surface.

FIGURE 4 is a representation of a non-limitative example of a measuring apparatus according to the invention, according to an exploded view.

Figure 4 illustrates a measuring apparatus 400 comprising an accessory 100 that may correspond to the accessory describes in figures 1-3, and a measuring unit 122. The measuring unit 122 is securely fixed to the accessory.

In the embodiment shown on FIGURE 4, the measuring unit 122 is a measurement plate. Of course the format of the measuring unit described is not limitative. The accessory 100 has dimensions that are comparable to those of the measuring unit 122, allowing it to seamlessly accommodate the measuring unit 122. As mentioned earlier, the measuring unit 122 is fastened to the accessory using screws that fit into the corresponding mounting holes 106.

When installing the measuring device 400, the measuring unit 122 is positioned on the front face of the accessory 100, specifically on the front face of the sheet 102 of the body. The second attachment means 106 of the different sheets of the body overlap when the sheets are assembled. The second attachment means 106 correspond to mounting holes. These mounting holes are designed to accommodate mounting elements such as screws, for example. The mounting elements are inserted through the mounting holes 106 of the main and intermediary sheets 102, 116, 120, and secured in place within the measuring unit 122. The mounting elements are attached to the rear surface of the accessory 100, particularly on the rear face of the sheet 120. As a result, the measuring unit 122 is securely fastened to the accessory 100 on both of its sides. The measuring unit 122 is configured to measure a force or a pressure applied to it. The measuring unit 122 is a device specifically engineered to quantify the magnitude of external forces acting upon it.

The measuring unit 122 may comprise, in a non-limiting way, at least one of the following :
- a communication module that permits to establish communication and exchange data with other devices or networks such as a computer, a Smartphone, or a server, etc.;
- at least one sensor that can detect and convert the applied force into a measurable output signal. This sensor can vary depending on the sensor's design and application. Common sensors include strain gauges, load cells, piezoelectric sensors, and force-sensitive resistors;
- at least one force sensor calibrated to accurately measure forces within a specific range. This range can vary widely depending on the sensor's intended purpose, from micro-newtons in delicate scientific experiments to kilonewtons in heavy industrial applications;
- at least one inertial sensor, for example including accelerometers and/or gyroscopes. Some advanced inertial sensors may also include magnetometers and barometers for additional functionality. Inertial sensor permits to capture and understand motion and orientation

changes. Inertial sensor enable an accurate measurement and tracking of movement.

The measuring unit 122 generates an output signal corresponding to the applied force by the user on. This signal can be analog (e.g., voltage or current) or digital, depending on the sensor's design. It provides a quantitative representation of the force magnitude for further analysis and interpretation. The output signal from the sensor is typically acquired using data acquisition systems or interfaces, which allow for recording and analysis of the force data. This data can be further processed, visualized, or used for control purposes, depending on the specific application.

The measuring unit 122 is designed to be easily mounted or integrated on the surface of the training machine where the force measurement is required.

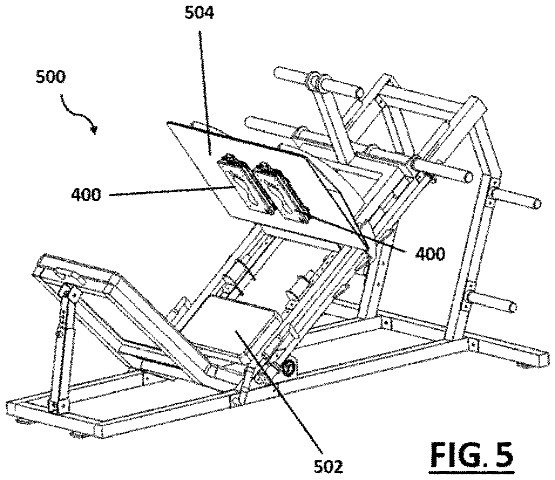

FIGURE 5 is a representation of a non-limitative example of a training machine equipped with a measuring apparatus according to the invention.

The training machine 500 represented on FIGURE 5 is a leg press.

The leg machine 500 comprises a seat 502 intended to be used by a user during the exercise, and a footplate 504 intended to be pushed by the legs of the user during the exercise.

As shown on FIGURE 5, two measuring apparatus 400, such as for example the measuring apparatus of FIGURE 4, are attached to the surface of the footplate 504 of the leg press machine 500.

In the embodiment shown on FIGURE 5, each measuring apparatus 400 is attached to the footplate 504 of the leg press machine 500 with first attachment means corresponding to magnets.

The user puts himself on the seat 502, and optionally adjusts the position of said seat 502 to be in a comfortable position that allows his knees to align with the footplate 504. The user places his feet on the measuring apparatus 400 fixed to the footplate 504. The user pushes against the measuring apparatus 400 by extending his legs while he applies a pressure to the measuring apparatus 400. Then, the user returns to the starting position by bending his knees and letting the footplate 504 go back to the starting position.

In some embodiments, the measuring apparatus 400 may be connected to a computer and/or a remote server. Ideally, this connection is established wirelessly for convenience. "Wireless" refers to various conventional and well-known methods of remotely connecting two devices, such as Bluetooth or Wi-Fi. This wireless connection allows for seamless data transmission and communication between the measuring apparatus and the computer/server.

More generally, the invention is not limited to the examples detailed above.

## Claims

1. Accessory (100) for equipping a training machine with a measuring unit (122), the accessory (100) comprising:
- a body with a rear face and a front face, said rear face being intended to bear against a surface of the training machine on which a user applies a pressure, said front face being intended to receive said measuring unit (122),
- at least one first attachment means (104, 105, 108, 118) for attaching said body to said training machine, and
- at least one second (106) attachment means for attaching said measuring unit (122) to said body;
so that when a user applies a pressure to said measuring unit (122), the pressure is transmitted to the surface of the training machine, the measuring unit (122) being able to measure at last one data relating to said pressure.

2. Accessory according to claim 1, wherein the at least one second attachment means (106) comprises at least one mounting hole to fix the measuring unit (122) on the front face of the body.

3. Accessory according to claim 2, wherein at least one mounting hole contains at least one flexible rubber attachment and at least one screw with a pre-set depth cut.

4. Accessory according to anyone of the preceding claims, wherein the at least one second attachment means (106) comprises at least one hole or thread provided in the body and intended to receive at least one mounting element for securing the body to the surface of the training machine if the latter is configured to receive said mounting element.

5. Accessory according to anyone of the preceding claims, wherein the at least one first attachment means (104) comprises at least one strap (110) equipping the body and provided to attach said body to the surface of the training machine.

6. Accessory according to anyone of the preceding claims, wherein the at least one first attachment means (108, 118) comprises at least one magnet arranged on the body, and more particularly on the rear face of the body.

7. Accessory according to anyone of the preceding claims, wherein the at least one first attachment means comprises at least one reusable double-sided adhesive arranged on the rear face of the body.

8. Accessory according to anyone of the preceding claims, wherein the at least one first attachment means (105) comprises at least one mounting peg, the at least one mounting peg intended to be inserted through the body and come to be attached on the surface of the training machine if the latter is configured to receive said mounting peg.

9. Accessory according to anyone of the preceding claims, wherein the at least one first attachment means (108,118) comprises at least one suction cup arranged on the body, and more particularly on the rear face of the body.

10. Accessory according to anyone of the preceding claims, wherein the body comprises a stacked assembly of at least two sheets (102, 120).

11. Accessory according to any of the claims 1 to 9, wherein the body comprises:
- a front sheet (102),
- a rear sheet (120), and
- at least one intermediary sheet (116), and in particular two intermediary sheets (116), stacked between said front (102) and rear (120) sheets.

12. Apparatus (400) comprising:
- at least one accessory (100) according to anyone of the claims 1 to 11, and
- at least one measuring unit (122) attached on the front face of the body of said accessory.

13. Apparatus (400) according to the preceding claim, wherein the measuring unit (122) comprises:
- a communication module for communicating with a distant computer/server, in a wireless or wired fashion;
- at least one force sensor,
- at least one inertial sensor.

14. A training machine comprising at least one accessory (100) according to anyone of the claims 1 to 11, the accessory (100) intended to be attached against a surface of the training machine.

15. Training machine comprising at least one measuring apparatus (400) according to anyone of the claims 12 or 13, the measuring apparatus (400) being configured to be attached on a surface of the training machine.

16. Training machine according to anyone of the claims 14 to 15, wherein the training machine is a leg press (500), and the accessory (100) is intended to be attached to a surface of said machine provided to be pushed by the leg(s) of a user.

## Amended claims

### Amended claims in accordance with Rule 137(2) EPC.

1. Accessory (100) for equipping a training machine with a measuring unit (122), the accessory (100) comprising:
- a body with a rear face and a front face, said rear face being configured to bear against a surface of the training machine on which a user applies a pressure, said front face being configured to receive said measuring unit (122),
- a plurality of attachment means (104, 105, 108, 118) for attaching said body to said training machine, and
- at least one second (106) attachment means for attaching said measuring unit (122) to said body;
so that when a user applies a pressure to said measuring unit (122), the pressure is transmitted to the surface of the training machine, the measuring unit (122) being configured to measure at last one data relating to said pressure.

2. Accessory according to claim 1, wherein the at least one second attachment means (106) comprises at least one mounting hole to fix the measuring unit (122) on the front face of the body.

3. Accessory according to claim 2, wherein at least one mounting hole contains at least one flexible rubber attachment and at least one screw with a pre-set depth cut.

4. Accessory according to anyone of the preceding claims, wherein the at least one second attachment means (106) comprises at least one hole or thread provided in the body and configured to receive at least one mounting element for securing the body to the surface of the training machine if the latter is configured to receive said mounting element.

5. Accessory according to anyone of the preceding claims, wherein the at least one first attachment means (104) comprises at least one strap (110) equipping the body and provided to attach said body to the surface of the training machine.

6. Accessory according to anyone of the preceding claims, wherein the at least one first attachment means (108, 118) comprises at least one magnet arranged on the body, and more particularly on the rear face of the body.

7. Accessory according to anyone of the preceding claims, wherein the at least one first attachment means comprises at least one reusable double-sided adhesive arranged on the rear face of the body.

8. Accessory according to anyone of the preceding claims, wherein the at least one first attachment means (105) comprises at least one mounting peg, the at least one mounting peg configured to be inserted through the body and come to be attached on the surface of the training machine if the latter is configured to receive said mounting peg.

9. Accessory according to anyone of the preceding claims, wherein the at least one first attachment means (108,118) comprises at least one suction cup arranged on the body, and more particularly on the rear face of the body.

10. Accessory according to anyone of the preceding claims, wherein the body comprises a stacked assembly of at least two sheets (102, 120).

11. Accessory according to any of the claims 1 to 9, wherein the body comprises:
- a front sheet (102),
- a rear sheet (120), and
- at least one intermediary sheet (116), and in particular two intermediary sheets (116), stacked between said front (102) and rear (120) sheets.

12. Apparatus (400) comprising:
- at least one accessory (100) according to anyone of the claims 1 to 11, and
- at least one measuring unit (122) attached on the front face of the body of said accessory.

13. Apparatus (400) according to the preceding claim, wherein the measuring unit (122) comprises:
- a communication module for communicating with a distant computer/server, in a wireless or wired fashion;
- at least one force sensor,
- at least one inertial sensor.

14. A training machine comprising at least one accessory (100) according to anyone of the claims 1 to 11, the accessory (100) configured to be attached against a surface of the training machine.

15. Training machine comprising at least one measuring apparatus (400) according to anyone of the claims 12 or 13, the measuring apparatus (400) being configured to be attached on a surface of the training machine.

16. Training machine according to anyone of the claims 14 to 15, wherein the training machine is a leg press (500), and the accessory (100) is configured to be attached to a surface of said machine provided to be pushed by the leg(s) of a user.
